# EUROPÄISCHE PATENTSCHRIFT

(11) **EP 1 343 752 B1**
(45) Veröffentlichungstag und Bekanntmachung des Hinweises auf die Patenterteilung: **02.05.2007**
(21) Anmeldenummer: 01270521.6
(22) Anmeldetag: 28.11.2001
(51) Int. Cl.: C07C 239/10

(54) **VERFAHREN ZUR HERSTELLUNG VON N-SUBSTITUIERTEN HYDROXYLAMINEN UND DEREN SALZEN**
METHOD FOR PRODUCING N-SUBSTITUTED HYDROXYLAMINES AND THE SALTS THEREOF
PROCEDE POUR PRODUIRE DES HYDROXYLAMINES N-SUBSTITUEES ET LEURS SELS

(30) Priorität: 11.12.2000 DE 10061623
(43) Veröffentlichungstag der Anmeldung: 17.09.2003
(73) Patentinhaber: Saltigo GmbH, 51369 Leverkusen (DE)
(72) Erfinder: DOCKNER, Michael, 50674 Köln (DE); EYMANN, Wolfgang, 51061 Köln (DE); KÖNIG, Bernd-Michael, 51467 Bergisch Gladbach (DE); HOLZEM, Helmut, 50829 Köln (DE)
(74) Vertreter: Wichmann, Birgid
(86) Internationale Anmeldenummer: PCT/EP2001/014051
(87) Internationale Veröffentlichungsnummer: WO 2002/048093

(56) Entgegenhaltungen:
- EP-A- 0 217 269
- WO-A-00/02848
- EMMOND, W D: "The preparation and properties of Oxaziranes" JOURNAL OF ORGANIC CHEMISTRY., Bd. 79, 1957, Seiten 5739-5754, XP002224392 AMERICAN CHEMICAL SOCIETY, WASHINGTON, DC., US ISSN: 0022-3263 in der Anmeldung erwähnt

## Beschreibung

Die vorliegende Erfindung betrifft ein Verfahren zur Herstellung von N-substituierten Hydroxylaminen aus N-substituierten Aryl- oder Heteroaryloxaziridinen durch saure Hydrolyse sowie die Isolierung der N-substituierten Hydroxylamine in Form ihrer Salze.

N-substituierte Hydroxylamine sowie insbesondere N-Alkyl-hydroxylamine und deren Salze dienen als Ausgangsverbindungen sowie Zwischenprodukte für die Synthese von Pharmawirkstoffen.

Aus J. Am. Chem. Soc. 79, 5749 (1957) ist es bereits bekannt, N-Alkyl-oxaziridine durch Säurehydrolyse in Aldehyde und N-Alkyl-hydroxylamine zu überführen. Beschrieben wird insbesondere die Hydrolyse von 2-tert.-Butyl-3-phenyloxaziridin in Gegenwart von 1,13 mol Schwefelsäure in Methanol. Nach 20 Stunden bei Raumtemperatur ist das 2-tert.-Butyl-3-phenyloxaziridin zu Benzaldehyd und N-(tert.-Butyl)hydroxylamin hydrolysiert. Zur Isolierung des Hydroxylamins wird der Reaktionsansatz zunächst mit Wasser versetzt. Der Benzaldehyd wird dann durch Extraktion mit Diethylether aus dem schwefelsauren Medium entfernt. Durch Zugabe von Natronlauge wird die wässrige Phase stark alkalisch gestellt und anschließend das N-(tert.-Butyl)hydroxylamin mit Hilfe von Diethylether kontinuierlich drei Tage lang aus der wässrigen Phase extrahiert. Nach Trocknen der organischen Phase und Entfernung des Lösungsmittels wird N-(tert.-Butyl)hydroxylamin mit einer Ausbeute von 82 % erhalten. Für ein technisches Verfahren ist eine Extraktionszeit von drei Tagen wenig geeignet und kann, wenn überhaupt, nur durch Verwendung einer speziellen Apparatur verkürzt werden. Weiterhin ergaben eigene Untersuchungen dieses Extraktionsvorgangs, dass aus einem stark alkalischen Medium (pH≥12) nur 20 % der theoretisch zu erwartenden Menge an Produkt mit Methylenchlorid extrahiert werden können.

Aus EP-A-0 217 269 ist bekannt, dass sich neben Schwefelsäure auch Salzsäure für die Hydrolyse von Oxaziridinen eignet. Schon nach einer Stunde Reaktionszeit ist die Umsetzung in diesem Fall vollständig. Der gebildete aromatische Aldehyd wird aus der sauren wäßrigen Phase mit Diethylether extrahiert. Anschließend gewinnt man das Hydrochlorid des N-substituierten-Hydroxylamins durch Aufkonzentrieren der wäßrigen Phase und Trocknen des Rückstands mit guten Ausbeuten. Wie thermische Untersuchungen zeigen, beginnt jedoch das Hydrochlorid sich bei Temperaturen oberhalb von 50°C zu zersetzen. Die höheren Temperaturen, die normalerweise zum Abdestillieren des Wassers benötigt werden, sind daher ungeeignet.

Für die Verwendung von N-Alkyl-substituierten Hydroxylaminen als Zwischenprodukte und/oder biologische Wirkstoffe ist es von Vorteil, diese in einer Form bereitzustellen, in der sie lagerstabil sind. Im allgemeinen sind insbesondere die N-Alkyl-substituierten Hydroxylamine hinsichtlich ihrer Lagerstabilität nicht befriedigend. Im Gegensatz dazu weisen die Salze der N-Alkyl-substituierten Hydroxylamine in der Regel eine wesentlich bessere Lagerstabilität auf, und ihre Isolierung ist daher von größerem Interesse. Wird das freie Hydroxylamin als Edukt benötigt, so kann es unmittelbar vor Verwendung aus den Salzen durch Zugabe von Base freigesetzt werden.

In der WO-A-00/02848 werden Carboxylate des N-(tert.-Butyl)hydroxylamins beschrieben. Zur Herstellung dieser Carboxylate wird zunächst 2-tert.-Butyl-3-phenyloxaziridin unter Verwendung von 1,5 Äquivalenten Schwefelsäure hydrolysiert. Selbst nach 20 Stunden Reaktionszeit werden jedoch im GC immer noch 5,8 Flächenprozent Edukt nachgewiesen (Example 4). Eine vollständige Umsetzung wird erst nach zwei weiteren Tagen beobachtet. Der gebildete Benzaldehyd wird, wie in den oben zitierten Fällen, durch Extraktion aus dem Rohgemisch entfernt. Im Unterschied zu den anderen literaturbekannten Verfahren besteht die weitere Vorgehensweise darin, dass man 1) das Reaktionsmedium mit einer niederen Carbonsäure RCOOH, wobei R Methyl, Ethyl, n-Propyl, i-Propyl, n-Butyl, i-Butyl oder t-Butyl ist, versetzt, 2) durch Zugabe von Natronlauge einen pH-Wert von ca. 5,5 einstellt, 3) anschließend aus dem schwach sauren Medium die Carboxylate des N-(tert.-Butyl)hydroxylamins mit Essigsäureethylester extrahiert und 4) die Carboxylate durch Kristallisation aus Essigsäureethylester bzw. Toluol, oder durch Destillation erhält. Bei der Herstellung von N-(tert.-Butyl)hydroxylammoniumacetat aus 2-tert.-Butyl-3-phenyloxaziridin werden auf diesem Weg nach einer Destillation und anschließender Kristallisation jedoch nur 45 % an gewünschtem Produkt erhalten. Wie die Differenz-Thermoanalyse z. B. des N-(tert.-Butyl)hydroxylammonium-acetats zeigt, beginnt sich das Salz bereits bei 70°C mit einer Wärmeproduktionsrate von 1 W/kg zu zersetzen. Die Entfernung des Essigsäureethylesters sollte deshalb nur im Vakuum erfolgen. Eine darüber hinausgehende thermische Belastung zur Reindestillation des N-(tert.-Butyl)hydroxylammoniumacetats ist somit nicht empfehlenswert.

Alle bekannten Methoden zur Herstellung von N-substituierten Hydroxylaminen und insbesondere N-Alkyl-Hydroxylaminen und deren Salzen durch saure Hydrolyse von Aryloxaziridinen in wässrigem Medium sind für eine Durchführung in technischem Maßstab hinsichtlich Reaktionsdauer, Isoliermethode, Extraktionszeiten und der verwendeten Lösungsmittel nicht befriedigend. Es besteht daher ein Bedürfnis nach einem wirtschaftlichen und sicherheitstechnisch unkritischem Verfahren zur Gewinnung von N-substituierten Hydroxylaminen und deren Salzen.

Gegenstand der Erfindung ist ein Verfahren zur Herstellung von N-substituierten Hydroxylaminen der allgemeinen Formel (I) und deren Salzen, 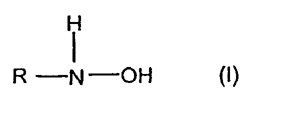 wobei
- R: einen Rest der allgemeinen Formel (II) 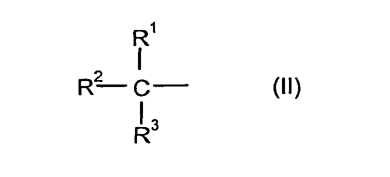
worin
- R¹, R² und R³: unabhängig voneinander für Wasserstoff, geradkettiges oder verzweigtes C₁-C₂₀-Alkyl, C₃-C₈-Cycloalkyl, geradkettiges oder verzweigtes C₂-C₁₀-Alkenyl oder C₆-C₁₀-Aryl stehen,
oder einen C₃-C₈-Cycloalkylrest darstellen kann,
durch saure Hydrolyse von Aryl- oder Heteroaryloxaziridinen der allgemeinen Formel (III) 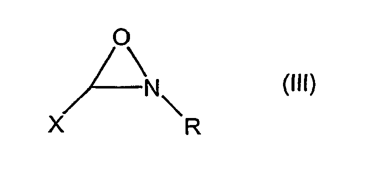 worin
- R: die zuvor für Formel (I) genannte Bedeutung hat, und
- X: einen C₆-C₁₂-Arylrest oder
einen C₄- oder C₅-Heteroarylrest darstellt,
unter Verwendung von mindestens zwei Äquivalenten einer Säure und eines mit Wasser mischbaren Lösungsmittels und
Isolierung der N-substituierten Hydroxylamine in Form ihrer Salze der allgemeinen Formel (IV) 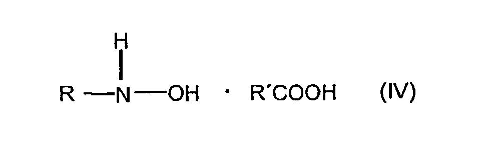 wobei R die zuvor genannte Bedeutung besitzt und R' Wasserstoff oder C₁-C₄-Alkyl bedeutet, indem das erhaltene Reaktionsgemisch den folgenden Schritten unterzogen wird
a) Abtrennung des bei der sauren Hydrolyse ebenfalls gebildeten aromatischen oder heteroaromatischen Aldehyds der Formel XCHO,
b) Einstellung des Reaktionsgemisches auf einen pH-Wert im Bereich von 7-11,
c) Abtrennung des N-substituierten Hydroxylamins der allgemeinen Formel (I) aus dem Reaktionsgemisch,
d) Zugabe einer Säure R'COOH zum N-substituierten Hydroxylamin der allgemeinen Formel (I) und
e) Isolierung des Salzes der allgemeinen Formel (IV).

Im erfindungsgemäßen Verfahren werden zunächst die Aryl- oder Heteroaryloxaziridine der allgemeinen Formel (III) hydrolytisch gespalten.

Für die saure Hydrolyse kommen als Säuren bevorzugt starke Säuren, insbesondere Salzsäure, Phosphorsäure, Schwefelsäure und Trifluoressigsäure in Frage. Bewährt hat sich vor allem Schwefelsäure.

Als mit Wasser mischbare Lösungsmittel kommen alle unter den Reaktionsbedingungen inerte, mit Wasser mischbare organische Lösungsmittel in Betracht, beispielsweise ein- und mehrwertige Alkohole mit bis zu 6 C-Atomen. Bevorzugt verwendet man Methanol oder Ethanol.

Sofern es sich im Rest R bei den Resten R¹, R² und/oder R³ um einen Alkylrest handelt, kann dieser wiederum substituiert sein durch gesättigte Cycloalkylreste, Arylreste, Alkinylreste und/oder Heteroatome, wie Halogen, Sauerstoff, Schwefel, Stickstoff und/oder Phosphoratome enthaltende Reste. Derartige gesättigte Cycloalkylreste können beispielsweise 3-12 C-Atome, derartige Arylreste beispielsweise 6-10 C-Atome und derartige Alkinylreste beispielsweise 2-6 C-Atome enthalten. Als Heteroatome enthaltende Reste kommen z.B. Fluor-, Chlor-, Brom-, Iod-, Hydroxy-, C₁-C₆-Alkoxy-, C₆-C₁₀-Phenoxy-, Carboxy-, C₁-C₆-Alkoxycarbonyl-, Nitro-, Amid-, Nitril-, Mercapto-, Sulfonyl-, Phosphit- und Phosphatgruppen in Frage.

Sofern es sich beim Rest R um einen Cycloalkylrest oder im Rest R bei den Resten R¹, R² und/oder R³ um einen Cycloalkyl, Aryl oder Alkenylrest handelt, so können diese substituiert sein beispielsweise durch C₁-C₆-Alkyl, Fluor, Chlor, Brom, Hydroxy, C₁-C₆-Alkoxy, Carboxy, C₁-C₆-Alkoxycarbonyl, Nitro, Sulfonyl und/oder Nitrilgruppen.

Besonders geeignet sind Aryl- oder Heteroaryloxaziridine der Formel (III), bei denen
- R: einen Rest der allgemeinen Formel (II) 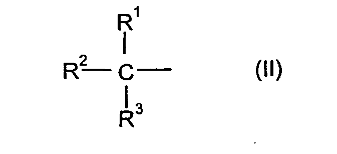
worin
- R¹, R² und R³: unabhängig voneinander für Wasserstoff, geradkettiges oder verzweigtes C₁-C₁₀-Alkyl, C₃-C₆-Cycloalkyl, geradkettiges oder verzweigtes C₂-C₆-Alkenyl oder Phenyl stehen,
oder einen C₃-C₆-Cycloalkylrest darstellt.

Besonders bevorzugt stehen die Reste R¹, R² und R³ unabhängig voneinander für Wasserstoff, Methyl, Ethyl, Propyl, iso-Propyl, Butyl, iso-Butyl, Pentyl, iso-Pentyl und Cyclopropyl. Bewährt hat sich aber genauso Nonadecyl.
- R: bedeutet bevorzugt n-Propyl, n-Butyl, iso-Butyl, t-Butyl, n-Pentyl, Cyclopropyl, Cyclopentyl oder Cyclohexyl.

Besonders geeignet sind ferner Aryl- oder Heteroaryloxaziridine der Formel (III), bei denen
- X: Phenyl, Naphthyl oder
einen Rest der Formeln (Va) oder (Vb) darstellen kann, 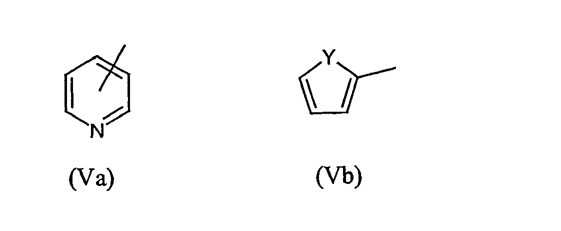 und Y für N, O oder S steht und sich die Bindung an den Oxaziridinring in o-, m- oder p-Stellung zum Stickstoff des Pyridinrings (Va) befinden kann.

Sofern X für einen Phenyl- oder Naphthylrest steht, so können diese substituiert sein beispielsweise durch C₁-C₆-Alkyl, Fluor, Chlor, Brom, Hydroxy, C₁-C₆-Alkoxy, C₁-C₆-Alkoxycarbonyl, Nitro, Sulfonyl und/oder Nitrilgruppen. Besonders steht X für einen 4-Methoxyphenyl-, 4-Methylphenyl- oder 4-Nitrophenylrest.

Bevorzugt lassen sich im erfindungsgemäßen Verfahren 2-tert.-Butyl-3-phenyl-oxaziridin in N-(tert.-Butyl)hydroxylamin und Benzaldehyd, 2-Isopropyl-3-(4-nitrophenyl)-oxaziridin und 2-Isopropyl-3-(4-methoxyphenyl)-oxaziridin in N-(Isopropyl)hydroxylamin und 4-Nitrobenzaldehyd bzw. 4-Methoxybenzaldehyd, 2-Cyclohexyl-3-(4-methylphenyl)-oxaziridin in N-(Cyclohexyl)hydroxylamin und 4-Methylbenzaldehyd, 2-Cyclopropyl-3-phenyloxaziridin in N-(Cyclopropyl)-hydroxylamin und Benzaldehyd und 2-Cyclopropylmethyl-3-phenyloxaziridin in N-(Cyclopropylmethyl)hydroxylamin und Benzaldehyd spalten.

Die im erfindungsgemäßen Verfahren eingesetzten Aryl- bzw. Heteroaryloxaziridine sind auf unterschiedlichen Synthesewegen zugänglich. In J. Am. Chem. Soc. 79, 5749 (1957) wird beispielsweise beschrieben, dass man Arylaldimine, die eine unsubstituierte oder eine mit einer Nitrogruppe substituierte Phenylgruppe enthalten, mit wasserfreier Peressigsäure in das entsprechende Oxaziridin überführen und durch Destillation isolieren kann. Aus EP-B-0 217 269 ist ferner bekannt, dass man zu N-Alkyl-substituierten Aryloxaziridinen durch Umsetzung von Arylaldiminen mit Perpropionsäure in Benzol gelangen kann. Auch m-Chlorperbenzoesäure kann zur Oxidation von Arylaldiminen verwendet werden (J. Chem. Soc. Perkin Trans 1 1990, 301 und 2390). Bekannt ist auch die Oxidation von Benzylidenaminosäureestern zu den entsprechenden Oxaziridinen mit Monoperphthalsäure in Diethylether als Lösungsmittel (Tetrahedron Lett. 28, 2453 (1994)).

Das erfindungsgemäße Verfahren wird üblicherweise so durchgeführt, dass man zunächst eine Mischung aus dem N-substituierten Aryl- oder Heteroaryloxaziridin und dem mit Wasser mischbaren Lösungsmittel herstellt. Die Konzentration des N-substituierten Aryl- oder Heteroaryloxaziridins in dem Lösungsmittel kann in sehr weiten Bereichen variiert werden und z.B. 5 bis 80 Gew.-% betragen, bevorzugt 20 bis 50 Gew.-%, insbesondere 35 bis 40 Gew.-%.

Zu der so erhaltenen Mischung wird eine Lösung der Säure in Wasser zugefügt. Die Dosiergeschwindigkeit wird dabei zweckmäßigerweise so gewählt, dass trotz der abzuführenden Reaktionswärme die Reaktionstemperatur 25°C nicht übersteigt. Die Untergrenze der Reaktionstemperatur kann beispielsweise bei 0°C liegen. Bevorzugt sind Reaktionstemperaturen im Bereich von 10 bis 25°C.

Bezogen auf 1 Mol N-substituiertes Aryl- oder Heteroaryloxaziridin müssen mindestens 2 Äquivalente Säure verwendet werden. Bevorzugt werden 2,0 bis 5,0 Äquivalente Säure, besonders bevorzugt 2,0 bis 2,5 Äquivalente Säure, insbesondere 2,1 bis 2,5 Äquivalente Säure eingesetzt. Die Konzentration der Säure in Wasser ist in weiten Bereichen variierbar und kann beispielsweise 10 bis 52 Gew.-% betragen. Bewährt hat sich beispielsweise 35-37%ige Salzsäure sowie 48-52%ige Schwefelsäure.

Nach beendeter Zugabe der Säure kann man die Reaktionsmischung bei z. B. 5 bis 30°C, bevorzugt 15 bis 25°C rühren, bis das Reaktionsende z.B. mittels Gaschromatographie festgestellt ist. Im allgemeinen wird bereits nach sehr kurzen Reaktionszeiten von drei bis vier Stunden kein Edukt mehr im GC-Chromatogramm nachgewiesen.

Neben der Bildung des gewünschten N-substituierten Hydroxylamins entsteht bei der Hydrolyse ein aromatischer oder heteroaromatischer Aldehyd der Formel XCHO. Im Fall der hydrolytischen Spaltung von 2-tert.-Butyl-3-phenyloxaziridin handelt es sich dabei um Benzaldehyd.

Nach Durchführung der sauren Hydrolyse wird dieser aromatische oder heteroaromatische Aldehyd aus dem Reaktionsgemisch abgetrennt (Schritt a). Zweckmäßigerweise erfolgt diese Abtrennung durch Extraktion. Möglich ist aber auch eine Destillation. Die Extraktion wird üblicherweise bei Raumtemperatur durchgeführt. Als Extraktionsmittel hat sich vor allem Methylenchlorid bewährt. Im Falle der vorhergehenden sauren Hydrolyse von N-substituierten Phenyloxaziridinen können durch zwei- bis dreimaliges Extrahieren mit jeweils einem Volumenäquivalent Methylenchlorid mehr als 95 % der theoretischen Menge des Benzaldehyds aus der wässrigen Phase entfernt werden. Neben dem Benzaldehyd können bei der sauren Hydrolyse auch ganz geringe Mengen des Dimethylacetals des Benzaldehyds gebildet werden. Dieses wird durch die Extraktion mit entfernt.

In Schritt b) wird das Reaktionsgemisch auf einen pH-Wert im Bereich von 7 bis 11 eingestellt. Dies erfolgt üblicherweise durch Zugabe einer wasserlöslichen Base. Bevorzugt wird das Reaktionsgemisch auf einen pH-Wert von 8-10 eingestellt, besonders bevorzugt von 8,5 - 9,5.

Als wasserlösliche Basen kommen prinzipiell Ammoniak, wasserlösliche primäre, sekundäre und tertiäre Amine und Alkali- und Erdalkalioxide, -hydroxide, -carbonate, -bicarbonate, -hydrogenphosphate und -phosphate in Frage. Bevorzugt verwendet man Natrium- oder Kaliumhydroxid.

Die wasserlösliche Base wird beispielsweise bei 0 bis 30 °C, bevorzugt bei 10 bis 25°C, zum Reaktionsgemisch zugegeben. Die Konzentration der Base ist beliebig variierbar und kann beispielsweise 10 bis 50 Gew.-% betragen. Bevorzugt sind 25 bis 45gew.-%ige Lösungen. Bewährt hat sich beispielsweise eine 28gew.-%ige Ammoniaklösung. Primäre, sekundäre und tertiäre Amine kann man auch als solche, d. h. ohne Wasser, einsetzen.

Bei der Zugabe der Base, wie z. B. eines Alkali- oder Erdalkalioxids, -hydroxids, -carbonats, -bicarbonats, -hydrogenphosphats oder -phosphats kann sich ein Feststoff bilden, den man leicht abfiltrieren kann. Wird bei der sauren Hydrolyse Schwefelsäure als Säure eingesetzt, so fällt bei der Zugabe von Natronlauge Natriumsulfat aus, das durch Filtration abgetrennt wird.

In Schritt c) erfolgt die Abtrennung des N-substituierten Hydroxylamins aus dem Reaktionsgemisch. Dies kann in Form zweier Alternativen c1) oder c2) erfolgen.

Bei Alternative c1) wird zunächst ein organisches Lösungsmittel zum Reaktionsgemisch zugegeben, das N-substituierte Hydroxylamin dadurch in die organische Lösungsmittelphase überführt und die das N-substituierte Hydroxylamin enthaltende organische Lösungsmittelphase anschließend abgetrennt.

Als organische Lösungsmittel können niedrig siedende, polare, mit Wasser nicht mischbare organische Lösungsmittel eingesetzt werden. Geeignet sind beispielsweise verzweigte und unverzweigte Alkylether wie tert.-Butylmethylether (Kp. 55-56°C), sowie halogenierte gesättigte Kohlenwasserstoffe wie Methylenchlorid (Kp. 39,8-40°C) und 1,2-Dichlorethan (Kp. 83°C). Bevorzugt verwendet man Methylenchlorid. Anschließend wird die organische Lösungsmittelphase abgetrennt, üblicherweise durch Extraktion. Im Fall der Herstellung von N-(tert.-Butyl)hydroxylamin aus 2-tert.-Butyl-3-phenyloxaziridin muss der Extraktionsvorgang aufgrund der vorhandenen Wasserlöslichkeit des N-(tert.-Butyl)hydroxylamins mehrfach wiederholt werden. Bei Verwendung von 2,7 Gewichtsteilen Methylenchlorid, bezogen auf eingesetztes 2-tert.-Butyl-3-phenyloxaziridin, sind fünf Extraktionen ausreichend. Es werden auf diesem Weg 70 bis 80 % N-(tert.-Butyl)hydroxylamin in die organische Phase überführt.

Bei Alternative c2) wird das N-substituierte Hydroxylamin aus dem Reaktionsgemisch herausdestilliert.

In Schritt d) erfolgt die Zugabe einer Säure R'COOH zum N-substituierten Hydroxylamin, wobei R' für Wasserstoff oder C₁-C₄-Alkyl steht. Bevorzugt steht R' für Methyl, Ethyl, n-Propyl, n-Butyl, Iso-Butyl oder tert- Butyl. Insbesondere steht R' für Methyl, d.h. es wird Essigsäure eingesetzt.

Wenn vor Schritt d) die Alternative c1) durchgeführt wurde, liegt das N-substituierte Hydroxylamin in einem organischen Lösungsmittel vor, bei Alternative c2) dagegen als solches.

Durch die Säurezugabe kommt es zur Bildung des gewünschten Salzes der allgemeinen Formel (IV). Vorab wird zweckmäßigerweise der Gehalt an N-(tert.-Butyl)-hydroxylamin in den vereinigten organischen Phasen durch Titration gegen Salzsäure bestimmt. Je nach gewünschtem Salz des N-substituierten Hydroxylamins werden 0,9 bis 1,2 Äquivalente der entsprechenden Säure R'COOH, bezogen auf den Gehalt an N-substituiertem Hydroxylamin in den vereinigten organischen Phasen, zugegeben. Bevorzugt liegt die verwendete Menge Säure bei 0,95 bis 1,05 Äquivalenten.

In Schritt e) wird das Salz des N-substituierten Hydroxylamins der allgemeinen Formel (IV) isoliert.

Hierzu wird die Reaktionsmischung nach der Säurezugabe üblicherweise zunächst auf ein Volumen eingeengt, in dem das Salz gerade noch löslich ist. Im allgemeinen werden hierzu 80 bis 95 % des ursprünglichen Volumens abdestilliert, bevorzugt 90 bis 93 %. Bei der Destillation muss wegen der thermischen Instablität der Salze des N-(tert.-Butyl)hydroxylamins darauf geachtet werden, dass die Manteltemperatur des Reaktors 50°C nicht übersteigt. Sollte das verwendete Lösungsmittel bei der maximalen Manteltemperatur noch nicht sieden, so muss unter einem entsprechend verminderten Druck gearbeitet werden.

Die anschließende Isolierung erfolgt bevorzugt durch Kristallisation aus einem Gemisch von Methylenchlorid und Cyclohexan.

Das Konzentrat kann man zur Ausfällung des Salzes bei 10 bis 30°C auf Cyclohexan geben. Bevorzugt wird das Salz bei 20 bis 25°C ausgefällt. Zur Vervollständigung der Niederschlagsbildung kann man auf 8 bis 12°C abkühlen. Der Niederschlag wird abfiltriert, mit Cyclohexan gewaschen und anschließend in einem schwachen Vakuum bei 15 bis 30°C, insbesondere bei 20 bis 25°C, getrocknet. Die bevorzugte Ausführungsvariante besteht darin, dass man bei 20 bis 25°C und 800±20 mbar einen schwachen Stickstoffstrom über das Salz bläst.

Die Aufarbeitung des Reaktionsgemisches über die Schritte a) - e) liefert das Salz des N-substituierten Hydroxylamins der allgemeinen Formel (IV) in einer Ausbeute von ca. 70 % der Theorie. Als Verunreinigung kann in sehr geringen Mengen das N-substituierte Aryl- oder Heteroarylnitron vorliegen, das sich beim Abdestillieren des Lösungsmittels aus Restmengen Benzaldehyd und N-substituiertem Hydroxylamin bildet. Im Fall der Herstellung von N-(tert.-Butyl)hydroxylamin handelt es sich hierbei um das tert.-Butyl-phenylnitron.

Das erfindungsgemäße Verfahren ist auf einfache Weise durchführbar, bei Einhaltung der Schritte a) - e) ergeben sich keine sicherheitstechnischen Probleme, das N-substituierte Hydroxylamin kann in dem angegebenen pH-Bereich auf effiziente Weise aus der wässrigen Phase extrahiert werden, die erzielbaren Ausbeuten sind sehr gut und Upscaling-Probleme sind ohne besonderen Aufwand zu lösen. Das Verfahren zeichnet sich ferner durch kurze Reaktionszeiten aus. Üblicherweise ist bereits nach maximal 4 Stunden die saure Hydrolyse vollständig erfolgt.

### Beispiele

### Beispiel 1

In einem 1 l Doppelmantelplanschliffbecherglas mit Bodenablass, Glasrührer, Temperaturfühler, Tropftrichter und Kühler werden 93,9 g 2-tert.-Butyl-3-phenyloxaziridin und 148,0 g Methanol bei Raumtemperatur vorgelegt. Zu dieser Lösung tropft man innerhalb von 30 Minuten 196,1 g einer 50 %igen Schwefelsäure, ohne dabei eine Innentemperatur von 25°C zu überschreiten. Die Reaktionsmischung wird bei 20 bis 25°C vier Stunden gerührt. Anschließend wird die schwefelsaure Lösung bei gleicher Temperatur dreimal mit jeweils 132 g Methylenchlorid extrahiert. Durch diese Extraktion kann mehr als 95 % des gebildeten Benzaldehyds wiedergewonnen werden. Der pH-Wert der schwefelsauren wässrigen Phase wird bei einer maximalen Innentemperatur von 30°C durch Zugabe von 45 %iger Natronlauge auf einen pH-Wert von 9 eingestellt. Der gebildete Niederschlag wird abfiltriert und einmal mit 251 g Methylenchlorid gewaschen. Die wässrige Phase wird einmal mit dem zum Waschen des abfiltrierten Natriumsulfats benutzten Methylenchlorids und noch viermal mit jeweils 251 g Methylenchlorid extrahiert. Der Gehalt an N-(tert.-Butyl)hydroxylamin in den vereinigten organischen Phasen wird durch Titration einer 2 g Probe gegen 1 M Salzsäure bestimmt. Die vereinigten organischen Phasen werden bei 20 bis 25°C mit 1,0 Äquivalenten Essigsäure versetzt. Bei einer maximalen Manteltemperatur von 50°C wird so viel Methylenchlorid abdestilliert, dass noch ca. 10 % des ursprünglichen Volumens in dem 1 l Doppelmantelplanschliffbecherglas zurückgeblieben sind. Unter starkem Rühren wird das gelbliche Konzentrat bei Raumtemperatur auf 160 g Cyclohexan, das vorher mit wenigen Impfkristallen versetzt wird, gegeben. Nach beendeter Zugabe wird die Suspension auf 10°C abgekühlt und das Produkt bei 8 bis 12°C abfiltriert. Der schwach gelb gefärbte Feststoff wird einmal in der Kälte mit 78 g Cyclohexan gewaschen und anschließend im Stickstoffstrom bei 20 bis 25°C und 800 mbar getrocknet. Es werden 53 g eines schwach gelb gefärbten Feststoffs erhalten. Dies entspricht einer Ausbeute von 70 % der Theorie an N-(tert.-Butyl)hydroxylammoniumacetat. Die gaschromatographische Analyse des isolierten Produktes ergibt:

| | |
|---|---|
| N-(tert.-Butyl)hydroxylamin: | 99,7 Flächen-% |
| N-tert.-Butyl-phenylnitron: | 0,3 Flächen-% |

## Patentansprüche

1. Verfahren zur Herstellung von N-substituierten Hydroxylaminen der allgemeinen Formel (I) und deren Salzen, 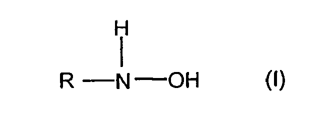 wobei
R einen Rest der allgemeinen Formel (II) 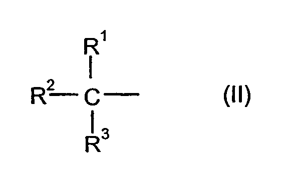
worin
R¹, R² und R³ unabhängig voneinander für Wasserstoff, geradkettiges oder verzweigtes C₁-C₂₀-Alkyl, C₃-C₈-Cycloalkyl, geradkettiges oder verzweigtes C₂-C₁₀-Alkenyl oder C₆-C₁₀-Aryl stehen,
oder einen C₃-C₈-Cycloalkylrest
darstellen kann,
durch saure Hydrolyse von Aryl- oder Heteroaryloxaziridinen der allgemeinen Formel (III) 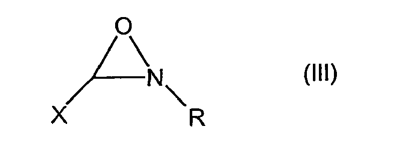 worin
R die zuvor für Formel (I) genannte Bedeutung hat, und
X einen C₆-C₁₂-Arylrest oder
einen C₄-C₅-Heteroarylrest darstellt,
unter Verwendung von mindestens zwei Äquivalenten einer Säure und eines mit Wasser mischbaren Lösungsmittels und
und Isolierung der N-substituierten Hydroxylamine in Form ihrer Salze der allgemeinen Formel (IV) 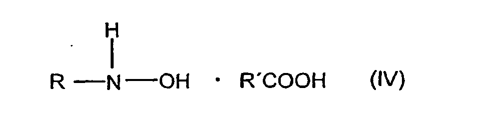 wobei R die zuvor genannte Bedeutung besitzt und R' Wasserstoff oder C₁-C₄-Alkyl bedeutet,
indem das erhaltene Reaktionsgemisch den folgenden Schritten unterzogen wird
a) Abtrennung des bei der sauren Hydrolyse ebenfalls gebildeten aromatischen oder heteroaromatischen Aldehyds der Formel XCHO,
b) Einstellung des Reaktionsgemisches auf einen pH-Wert im Bereich von 7-11,
c) Abtrennung des N-substituierten Hydroxylamins
d) Zugabe einer Säure R'COOH zum N-substituierten Hydroxylamin und
e) Isolierung des Salzes der allgemeinen Formel (IV).

2. Verfahren nach Anspruch 1, **dadurch gekennzeichnet, dass** als Säuren Salzsäure, Phosphorsäure, Schwefelsäure und Trifluoressigsäure eingesetzt werden.

3. Verfahren nach Anspruch 1 oder 2, **dadurch gekennzeichnet, dass** als mit Wasser mischbare Lösungsmittel ein- und mehrwertige Alkohole mit bis zu 6 C-Atomen, bevorzugt Methanol oder Ethanol eingesetzt werden.

4. Verfahren nach einem oder mehreren der Ansprüche 1-3, **dadurch gekennzeichnet, dass** Aryl- oder Heteroaryloxaziridine der Formel (III) eingesetzt werden, bei denen
R¹, R² und R³ unabhängig voneinander für Wasserstoff, geradkettiges oder verzweigtes C₁-C₁₀-Alkyl, C₃-C₆-Cycloalkyl, geradkettiges oder verzweigtes C₂-C₆-Alkenyl oder Phenyl stehen,
oder R einen C₃-C₆-Cycloalkylrest darstellt.

5. Verfahren nach einem oder mehreren der Ansprüche 1-4, **dadurch gekennzeichnet, dass** die Reste R¹, R² und R³ in Formel (II) unabhängig voneinander für Wasserstoff, Methyl, Ethyl, Propyl, iso-Propyl, Butyl, iso-Butyl, Pentyl, iso-Pentyl oder Cyclopropyl stehen.

6. Verfahren nach einem oder mehreren der Ansprüche 1-5, **dadurch gekennzeichnet, dass** der Rest R n-Propyl, n-Butyl, iso-Butyl, t-Butyl, n-Pentyl, Cyclopropyl, Cyclopentyl, Cyclohexyl bedeutet.

7. Verfahren nach einem oder mehreren der Ansprüche 1-6, **dadurch gekennzeichnet, dass** Aryl- oder Heteroaryloxaziridine der Formel (III) eingesetzt werden, bei denen
X Phenyl, Naphthyl oder
einen Rest der Formeln (Va) oder (Vb) darstellen kann, 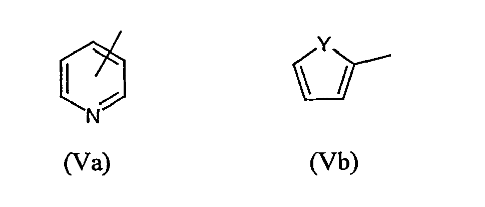 und Y für N, O oder S steht und sich die Bindung an den Oxaziridinring in o-, m- oder p-Stellung zum Stickstoff des Pyridinrings (Va) befinden kann.

8. Verfahren nach einem oder mehreren der Ansprüche 1-7, **dadurch gekennzeichnet, dass** 2-tert.-Butyl-3-phenyloxaziridin in N-(tert.-Butyl)hydroxylamin und Benzaldehyd, 2-Isopropyl-3-(4-nitrophenyl)-oxaziridin und 2-Isopropyl-3-(4-methoxyphenyl)-oxaziridin in N-(Isopropyl)hydroxylamin und 4-Nitrobenzaldehyd bzw. 4-Methoxybenzaldehyd, 2-Cyclohexyl-3-(4-methylphenyl)-oxaziridin in N-(Cyclohexyl)hydroxylamin und 4-Methylbenzaldehyd, 2-Cyclopropyl-3-phenyloxaziridin in N-(Cyclopropyl)hydroxylamin und Benzaldehyd und 2-Cyclopropylmethyl-3-phenyloxaziridin in N-(Cyclopropylinethyl)hydroxylamin und Benzaldehyd gespalten wird.

9. Verfahren nach einem oder mehreren der Ansprüche 1-8, **dadurch gekennzeichnet, dass** bezogen auf 1 Mol N-substituiertes Aryl- oder Heteroaryloxaziridin 2,0 bis 5,0, bevorzugt 2,0 bis 2,5 Äquivalente Säure eingesetzt werden.

10. Verfahren nach einem oder mehreren der Ansprüche 1-10, **dadurch gekennzeichnet, dass** in Schritt b) das Reaktionsgemisch durch Zugabe einer wasserlöslichen Base auf einen pH-Wert im Bereich von 8-10, bevorzugt von 8,5 - 9,5 eingestellt wird.

11. Verfahren nach einem oder mehreren der Ansprüche 1-10, **dadurch gekennzeichnet, dass** als wasserlösliche Basen Ammoniak, wasserlösliche primäre, sekundäre und tertiäre Amine und Alkali- und Erdalkalioxide, -hydroxide, -carbonate, -bicarbonate, -hydrogenphosphate und -phosphate, bevorzugt Natrium- oder Kaliumhydroxid eingesetzt werden.

12. Verfahren nach einem oder mehreren der Ansprüche 1-11, **dadurch gekennzeichnet, dass** in Schritt c) ein organisches Lösungsmittel zum Reaktionsgemisch zugegeben, das N-substituierte Hydroxylamin **dadurch** in die organische Lösungsmittelphase überführt und die das N-substituierte Hydroxylamin enthaltende organische Lösungsmittelphase anschließend abgetrennt wird.

13. Verfahren nach Anspruch 12, **dadurch gekennzeichnet, dass** als organisches Lösungsmittel ein niedrig siedendes, polares, mit Wasser nicht mischbare organisches Lösungsmittel eingesetzt wird, bevorzugt verzweigte und unverzweigte Alkylether, insbesondere tert.-Butylmethylether, oder halogenierte gesättigte Kohlenwasserstoffe, insbesondere Methylenchlorid oder 1,2-Dichlorethan.

14. Verfahren nach einem oder mehreren der Ansprüche 1-13, **dadurch gekennzeichnet, dass** in Schritt d) eine Säure R'COOH zur organischen Lösungsmittelphase zugegeben wird, wobei R' für Methyl, Ethyl, n-Propyl, n-Butyl, Iso-Butyl oder tert- Butyl steht.

15. Verfahren nach einem oder mehreren der Ansprüche 1-14, **dadurch gekennzeichnet, dass** 0,9 bis 1,2 Äquivalente, bevorzugt 0,95 bis 1,05 Äquivalente der Säure R'COOH, bezogen auf den Gehalt an N-substituiertem Hydroxylamin im Reaktionsgemisch zugegeben werden.

16. Verfahren nach einem oder mehreren der Ansprüche 1-15, **dadurch gekennzeichnet, dass** in Schritt e) das Salz des N-substituierten Hydroxylamins der allgemeinen Formel (IV) durch Kristallisation aus einem Gemisch von Methylenchlorid und Cyclohexan isoliert wird.

## Claims

1. Process for preparing N-substituted hydroxylamines of the general formula (I) and salts thereof, 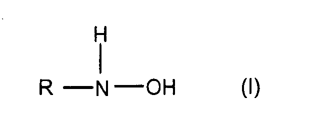 where
R may represent a radical of the general formula (II) 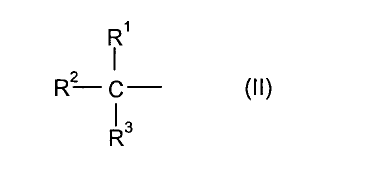
in which
R¹, R² and R³ independently of one another represent hydrogen, straight-chain or branched C₁-C₂₀-alkyl, C₃-C₈-cycloalkyl, straight-chain or branched C₂-C₁₀-alkenyl or C₆-C₁₀-aryl,
or a C₃-C₈-cycloalkyl radical,
by acid hydrolysis of aryl- or heteroaryloxaziridines of the general formula (III) 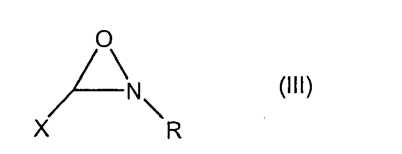 in which
R has the meaning given above for formula (I) and
X represents a C₆-C₁₂-aryl radical or
a C₄-C₅-heteroaryl radical,
using at least two equivalents of an acid and a water-miscible solvent and
isolation of the N-substituted hydroxylamines in the form of their salts of the general formula (IV) 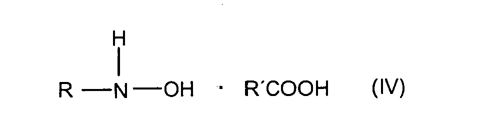 where R has the meaning given above and R' represents hydrogen or C₁-C₄-alkyl,
by subjecting the resulting reaction mixture to the following steps
a) removal of the aromatic or heteroaromatic aldehyde of the formula XCHO which is also formed during the acid hydrolysis,
b) adjustment of the reaction mixture to a pH in the range of 7-11,
c) removal of the N-substituted hydroxylamine
d) addition of an acid R'COOH to the N-substituted hydroxylamine and
e) isolation of the salt of the general formula (IV).

2. Process according to Claim 1, **characterized in that** the acids used are hydrochloric acid, phosphoric acid, sulphuric acid and trifluoroacetic acid.

3. Process according to Claim 1 or 2, **characterized in that** the water-miscible solvents used are mono- and polyhydric alcohols having up to 6 carbon atoms, preferably methanol or ethanol.

4. Process according to one or more of Claims 1-3, **characterized in that** aryl- or heteroaryloxaziridines of the formula (III) are used in which
R¹, R² and R³ independently of one another represent hydrogen, straight-chain or branched C₁-C₁₀-alkyl, C₃-C₆-cycloalkyl, straight-chain or branched C₂-C₆-alkenyl or phenyl,
or R represents a C₃-C₆-cycloalkyl radical.

5. Process according to one or more of Claims 1-4, **characterized in that** the radicals R¹, R² and R³ in formula (II) independently of one another represent hydrogen, methyl, ethyl, propyl, isopropyl, butyl, isobutyl, pentyl, isopentyl or cyclopropyl.

6. Process according to one or more of Claims 1-5, **characterized in that** the radical R represents n-propyl, n-butyl, isobutyl, t-butyl, n-pentyl, cyclopropyl, cyclopentyl, cyclohexyl.

7. Process according to one or more of Claims 1-6, **characterized in that** aryl- or heteroaryloxaziridines of the formula (III) are used in which
X may represent phenyl, naphthyl or
a radical of the formula (Va) or (Vb) 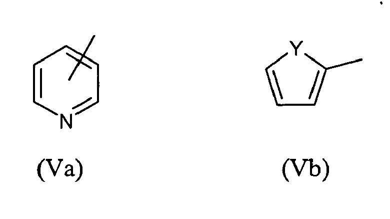 and Y represents N, O or S and the bond to the oxaziridine ring may be located in a position ortho, meta or para to the nitrogen of the pyridine ring (Va).

8. Process according to one or more of Claims 1-7, **characterized in that** 2-tert-butyl-3-phenyloxaziridine is cleaved into N-(tert-butyl)hydroxylamine and benzaldehyde, 2-isopropyl-3-(4-nitrophenyl)-oxaziridine and 2-isopropyl-3-(4-methoxyphenyl)-oxaziridine are cleaved into N-(isopropyl)hydroxylamine and 4-nitrobenzaldehyde and 4-methoxybenzaldehyde, respectively, 2-cyclohexyl-3-(4-methylphenyl)-oxaziridine is cleaved into N-(cyclohexyl)hydroxylamine and 4-methylbenzaldehyde, 2-cyclopropyl-3-phenyloxaziridine is cleaved into N-(cyclopropyl)hydroxylamine and benzaldehyde and 2-cyclopropylmethyl-3-phenyloxaziridine is cleaved into N-(cyclopropylmethyl)hydroxylamine and benzaldehyde.

9. Process according to one or more of Claims 1-8, **characterized in that**, based on 1 mol of N-substituted aryl- or heteroaryloxaziridine, from 2.0 to 5.0, preferably from 2.0 to 2.5, equivalents of acid are used.

10. Process according to one or more of Claims 1-10, **characterized in that** in step b) the reaction mixture is adjusted by addition of a water-soluble base to a pH in the range of 8 - 10, preferably 8.5 - 9.5.

11. Process according to one of more of Claims 1-10, **characterized in that** the water-soluble bases used are ammonia, water-soluble primary, secondary and tertiary amines and alkali metal and alkaline earth metal oxides, hydroxides, carbonates, bicarbonates, hydrogenphosphates and phosphates, preferably sodium hydroxide or potassium hydroxide.

12. Process according to one or more of Claims 1-11, **characterized in that** in step c) an organic solvent is added to the reaction mixture, which results in the N-substituted hydroxylamine passing into the organic solvent phase, and the organic solvent phase which contains the N-substituted hydroxylamine is then separated off.

13. Process according to Claim 12, **characterized in that** the organic solvent used is a low-boiling, polar, water-immiscible organic solvent, preferably a branched or unbranched alkyl ether, in particular tert-butyl methyl ether, or a halogenated saturated hydrocarbon, in particular methylene chloride or 1,2-dichloroethane.

14. Process according to one or more of Claims 1-13, **characterized in that** in step d) an acid R'COOH is added to the organic solvent phase, where R' represents methyl, ethyl, n-propyl, n-butyl, isobutyl or tert butyl.

15. Process according to one or more of Claims 1-14, **characterized in that** from 0.9 to 1.2 equivalents, preferably from 0.95 to 1.05 equivalents, of the acid R'COOH are added, based on the amount of N-substituted hydroxylamine in the reaction mixture.

16. Process according to one or more of Claims 1-15, **characterized in that** in step e) the salt of the N-substituted hydroxylamine of the general formula (IV) is isolated by crystallization from a mixture of methylene chloride and cyclohexane.

## Revendications

1. Procédé de préparation d'hydroxylamines N-substituées de formule générale (I) et de leurs sels 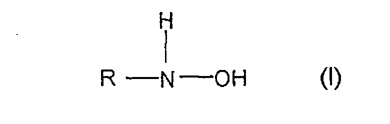 dans laquelle :
R peut représenter un radical de formule générale (II) 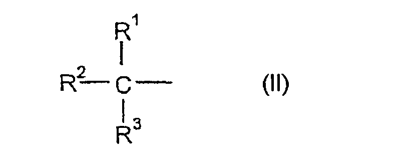
dans laquelle :
R¹, R² et R³ peuvent représenter, indépendamment les uns des autres, un atome d'hydrogène, un groupe alkyle en C₁-C₂₀ linéaire ou ramifié, un groupe cycloalkyle en C₃-C₈, un groupe alcényle en C₂-C₁₀ linéaire ou ramifié ou un groupe aryle en C₆-C₁₀,
ou un radical cycloalkyle en C₃-C₈, par l'hydrolyse acide d'aryl- ou d'hétéroaryloxaziridines de formule générale (III) 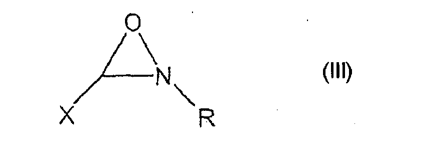 dans laquelle :
R présente la signification mentionnée précédemment pour la formule (I), et
X représente
un radical aryle en C₆-C₁₂, ou
un radical hétéroaryle en C₄-C₅,
en utilisant au moins deux équivalents d'un acide et d'un solvant miscible à l'eau, et
l'isolation des hydroxylamines N-substituées sous la forme de leurs sels de formule générale (IV) 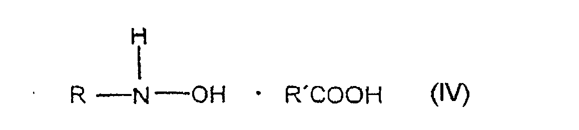 dans laquelle R présente la signification mentionnée précédemment et R' représente un atome d'hydrogène ou un groupe alkyle en C₁-C₄,
en ce que le mélange réactionnel obtenu est soumis aux étapes suivantes :
a) la séparation de l'aldéhyde aromatique ou hétéroaromatique de formule XCHO, également formé lors de l'hydrolyse acide,
b) l'ajustement du mélange réactionnel à un pH dans la plage de 7 à 11,
c) la séparation de l'hydroxylamine N-substituée,
d) l'addition d'un acide R'COOH à l'hydroxylamine N-substituée, et
e) l'isolation du sel de formule générale (IV).

2. Procédé selon la revendication 1, **caractérisé en ce que** l'on utilise, en tant qu'acides, l'acide chlorhydrique, l'acide phosphorique, l'acide sulfurique et l'acide trifluoroacétique.

3. Procédé selon la revendication 1 ou 2, **caractérisé en ce que** l'on utilise, en tant que solvant miscible à l'eau, des alcools monohydriques et polyhydriques renfermant jusqu'à 6 atomes de carbone, de préférence le méthanol ou l'éthanol.

4. Procédé selon l'une ou plusieurs des revendications 1 à 3, **caractérisé en ce que** l'on utilise des aryl- ou hétéroaryloxaziridines de formule (III), dans lesquelles :
R¹, R² et R³ représentent, indépendamment les uns des autres, un atome d'hydrogène, un groupe alkyle en C₁-C₁₀ linéaire ou ramifié, un groupe cycloalkyle en C₃-C₆, un groupe alcényle en C₂-C₆ linéaire ou ramifié ou un groupe phényle,
ou R représente un radical alkyle en C₃-C₆.

5. Procédé selon l'une ou plusieurs des revendications 1 à 4, **caractérisé en ce que** les radicaux R¹, R² et R³ dans la formule (II) représentent, indépendamment les uns des autres, un atome d'hydrogène, un groupe méthyle, un groupe éthyle, un groupe propyle, un groupe isopropyle, un groupe butyle, un groupe isobutyle, un groupe pentyle, un groupe isopentyle ou un groupe cyclopropyle.

6. Procédé selon l'une ou plusieurs des revendications 1 à 5, **caractérisé en ce que** le radical R représente un groupe n-propyle, un groupe n-butyle, un groupe isobutyle, un groupe t-butyle, un groupe n-pentyle, un groupe cyclopropyle, un groupe cyclopentyle ou un groupe cyclohexyle.

7. Procédé selon l'une ou plusieurs des revendications 1 à 6, **caractérisé en ce que** l'on utilise des aryl- ou hétéroaryloxaziridines de formule (III), dans lesquelles :
X peut représenter un groupe phényle, un groupe naphtyle, ou
un radical de formule (Va) ou (Vb) 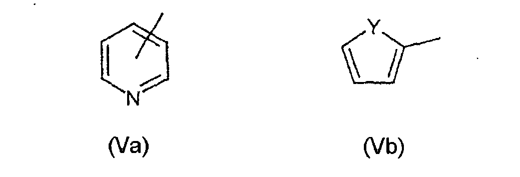 et
Y représente un atome d'azote, un atome d'oxygène ou un atome de soufre, et la liaison au noyau oxaziridine peut se trouver en position o-, m-ou p- par rapport à l'atome d'azote du noyau pyridine (Va).

8. Procédé selon l'une ou plusieurs des revendications 1 à 7, **caractérisé en ce que** la 2-tert-butyl-3-phényloxaziridine est clivée en N-(tert-butyl)hydroxylamine et benzaldéhyde, la 2-isopropyl-3-(4-nitrophényl)oxaziridine et la 2-isopropyl-3-(4-méthoxyphényl)oxaziridine sont clivées en N-(isopropyl)hydroxylamine et 4-nitrobenzaldéhyde ou 4-méthoxybenzaldéhyde, respectivement, la 2-cyclohexyl-3-(4-méthylphényl)oxaziridine est clivée en N-(cyclohexyl)hydroxylamine et 4-méthylbenzaldéhyde, la 2-cyclopropyl-3-phényloxaziridine est clivée en N-(cyclopropyl)hydroxylamine et benzaldéhyde, et la 2-cyclopropylméthyl-3-phényloxaziridine est clivée en N-(cyclopropylméthyl)hydroxylamine et benzaldéhyde.

9. Procédé selon l'une ou plusieurs des revendications 1 à 8, **caractérisé en ce que** l'on utilise de 2,0 à 5,0, de préférence de 2,0 à 2,5 équivalents d'acide par rapport à 1 mol d'aryl- ou d'hétéroaryloxaziridine N-substituée.

10. Procédé selon l'une ou plusieurs des revendications 1 à 10, **caractérisé en ce que**, dans l'étape b), le mélange réactionnel est ajusté à un pH dans la plage de 8 à 10, de préférence de 8,5 à 9,5, par l'addition d'une base hydrosoluble.

11. Procédé selon l'une ou plusieurs des revendications 1 à 10, **caractérisé en ce que** l'on utilise, en tant que bases hydrosolubles, l'ammoniac, des amines primaires, secondaires et tertiaires hydrosolubles, et des oxydes, des hydroxydes, des carbonates, des bicarbonates, des hydrogénophosphates et des phosphates de métaux alcalins et de métaux alcalino-terreux, de préférence l'hydroxyde de sodium ou de potassium.

12. Procédé selon l'une ou plusieurs des revendications 1 à 11, **caractérisé en ce que**, dans l'étape c), un solvant organique est ajouté au mélange réactionnel, l'hydroxylamine N-substituée est ainsi transférée dans la phase solvant organique, et la phase solvant organique contenant l'hydroxylamine N-substituée est ensuite séparée.

13. Procédé selon la revendication 12, **caractérisé en ce que** l'on utilise, en tant que solvant organique, un solvant organique polaire, à faible point d'ébullition, immiscible à l'eau, de préférence des éthers alkyliques ramifiés et non-ramifiés, en particulier l'éther tert-butylméthylique, ou des hydrocarbures saturés halogénés, en particulier le chlorure de méthylène ou le 1,2-dichloroéthane.

14. Procédé selon l'une ou plusieurs des revendications 1 à 13, **caractérisé en ce que**, dans l'étape d), un acide R'COOH est ajouté à la phase solvant organique, dans lequel R' représente un groupe méthyle, un groupe éthyle, un groupe n-propyle, un groupe n-butyle, un groupe isobutyle ou un groupe tert-butyle.

15. Procédé selon l'une ou plusieurs des revendications 1 à 14, **caractérisé en ce que**, de 0,9 à 1,2 équivalent, de préférence 0,95 à 1,05 équivalent de l'acide R'COOH par rapport à la teneur en hydroxylamine N-substituée, est ajouté dans le mélange réactionnel.

16. Procédé selon l'une ou plusieurs des revendications 1 à 15, **caractérisé en ce que**, dans l'étape e), le sel de l'hydroxylamine N-substituée de formule générale (IV) est isolé par cristallisation dans un mélange de chlorure de méthylène et de cyclohexane.
